(19)

**Europäisches Patentamt**

**European Patent Office**

**Office européen des brevets**

(11) **EP 3 536 354 A1**

(12) **EUROPEAN PATENT APPLICATION**

(43) Date of publication:
**11.09.2019 Bulletin 2019/37**

(21) Application number: **18159979.6**

(22) Date of filing: **05.03.2018**

(51) Int Cl.:
*A61L 27/50* (2006.01)          *A61L 27/56* (2006.01)
*G09B 23/28* (2006.01)          *G09B 23/30* (2006.01)
*G09B 23/34* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Universität Zürich
8006 Zürich (CH)**

(72) Inventors:
• **FARSHAD, Mazda
  8126 Zumikon (CH)**
• **BURKHARD, Marco
  8833 Samstagern (CH)**
• **FÜRNSTAHL, Philipp
  8193 Eglisau (CH)**

(74) Representative: **Schulz Junghans
Patentanwälte PartGmbB
Großbeerenstraße 71
10963 Berlin (DE)**

(54) **SYNTHETIC BONE AND METHOD FOR MANUFACTURING A SYNTHETIC BONE USING RAPID PROTOTYPING**

(57)     The invention relates to a synthetic mammalian bone (1), wherein the synthetic bone (1) comprises at least one compound that is suitable for rapid prototyping, wherein the synthetic bone (1) comprises a cortical bone portion (3) and a cancellous bone portion (2), wherein the cortical bone portion (3) forms a layer enclosing the cancellous bone portion (2), wherein the cortical bone portion (3) exhibits a higher density than the cancellous bone portion (2), wherein the cancellous bone portion (2) is formed as a periodic pattern (6).

The invention further relates to a method for manufacturing a synthetic bone (1).

**EP 3 536 354 A1**

**Description**

The invention relates to a synthetic bone and a method for manufacturing the synthetic bone with rapid prototyping.

Synthetic bones for surgical training and preoperative simulation of spine surgeries are known in the state of the art.

Synthetic bones resemble their real counterparts in many aspects. For surgery the synthetic bones should particularly exhibit properties as close as possible to their real counterparts. These properties particularly comprise haptic, physical and biomechanical properties such as weight, dimension, shape, hardness, porosity etc. Furthermore, it is advantageous if also the acoustic properties are similar. Of particular interest can be the torque required to insert surgical screws, as during simulation of surgery also surgical screws are used.

WO 2016115625 A1 teaches an artificial bone with a cancellous bone portion comprising at least one fiber component, a liquid and a binder. The resulting cancellous bone portion exhibits similar properties to their real counterpart, while particularly having similar radiodensity characteristics.

The artificial bone comprises a plurality of components and requires a dedicated manufacturing process.

An object of the present invention is to provide a synthetic bone that exhibits similar properties to a real bone as well as a method for providing the synthetic bone. This is achieved by a synthetic bone having the features of claim 1.

Advantageous embodiments are described in the subclaims.

According to claim 1, a synthetic mammalian, particularly a synthetic human bone, mimicking a mammalian, particularly human bone, consists of or comprises at least one compound that is suitable for rapid prototyping, particularly 3D-printing, wherein the at least one compound is particularly UV-curable, wherein the synthetic bone comprises or consists of a cortical bone portion and a cancellous bone portion, wherein the cortical bone portion encloses, particularly completely encloses the cancellous bone portion, wherein the cortical bone portion exhibits a higher density than the cancellous bone portion, wherein the cancellous bone portion of the synthetic bone is formed as a periodic pattern.

The cortical bone portion and the cancellous bone portion can comprise or consist of the same compound.

The synthetic bone is particularly made by a rapid prototyping method, particularly by a 3D-printing method, wherein the synthetic bone is manufactured from at the least one compound used during the rapid prototyping process.

A compound that is suitable for rapid prototyping is particularly a compound that is inter-connectable, curable, polymerizable, or otherwise chemically or physically connectable on a molecular level. The compound in the synthetic bone is interconnected according to one of the laid-out principles.

The synthetic bone therefore is manufactured by a rapid prototyping method such as 3D-printing.

The term rapid prototyping refers particularly to a 3D-printing method, a fused deposition modeling method, a multi jet modelling method, or a selective laser sintering method.

The synthetic bone particularly exhibits at least one of the following similar or identical properties to a real bone:

- physical properties, such as shape, weight, and/or size;
- haptic properties, such as touch and feel, or the friction coefficient of the cortical and the cancellous bone portion;
- mechanical properties such as, breakage, stiffness, hardness, and/or other mechanical properties;
- acoustic properties, such as sound damping properties;
- insertional torque properties, such as for example for driving a surgical screw through the cortical bone portion into the cancellous bone portion.

At least some of the above listed properties should be closely matched to a real bone in order to provide a suitable model for a simulated surgery.

The synthetic bone particularly differs from a real bone by its composition and by the periodic pattern that represents the cancellous bone portion.

The cortical bone portion corresponds to the cortex of a bone and therefore forms a cortical layer around the cancellous bone portion.

The periodic cancellous bone portion of the synthetic bone is particularly arranged at the same place as the cancellous bone portion of a real bone or the bone model.

By adjusting the thickness of the cortical bone portion and/or by adjusting the periodical pattern forming the cancellous bone portion, different kinds of bones with different physical properties can be mimicked.

It is thus possible to provide a synthetic bone with osteoporotic properties or a synthetic bone that resembles a bone with a typical or an average hardness (in the following also referred to as an average bone), i.e. a hardness of bones of a healthy adult person or even a synthetic bone whose hardness and insertional torque properties mimic a particularly hard bone.

The periodic pattern can be naturally expanded or reduced in thickness, density, periodicity, size and/or volume in order to match certain properties of the real counterpart bone.

The periodicity of the periodic pattern allows for a natural reduction or expansion of the cancellous bone portion by reducing or increasing the number of periods along one, two or three dimensions such that the periodic pattern completely fills the cancellous bone portion of the synthetic bone.

The use of a periodic pattern for the cancellous bone portion is particularly advantageous as it allows the provision of synthetic bones with varying degrees of cancellous bone portion hardness and elasticity. The cancellous bone portion can be adapted easily by e.g. adapting the periodicity or density of the periodic pattern or by altering a wall thickness of the periodic pattern. This way, the synthetic bone can exhibit bone properties corresponding to various kinds of real bones.

Another advantage of the bone according to the invention is that a variety of different bones, including various physical properties, such as hardness etc., can be manufactured by reducing the complexity of a real bone structure, particularly the complexity of the cancellous bone structure to a periodic pattern and defining the thickness of the cortical layer of the bone.

This allows for a parametrization of the synthetic bone with few parameters, such as the cortical bone portion thickness, the periodicity of the cancellous bone portion, and/or the thickness of the walls forming the periodic pattern of the cancellous bone portion.

By adjusting any of these parameters, the physical, particularly the mechanical properties of the synthetic bone can be altered in a simple and parametrized manner.

The parameters can be derived from patient data, particularly from diagnosed bone properties, such as osteoporosis.

As many periodic patterns can be analytically described, the manufacturing of the synthetic bone is particularly suitable for implementation in software configured for providing models or file formats for rapid prototyping, particularly 3D-printing.

Also, due to the rapid prototyping process a well-defined manufacturing result of the cancellous bone portion is granted as compared to cancellous bone portions that are formed from mixtures comprising fiber-compounds that are not manufactured by rapid prototyping and that are not suited for rapid prototyping, particularly 3D-printing.

Furthermore, the periodic pattern can be spatially continued arbitrarily without a connecting mismatch or a discontinuity that might occur when using other non-periodic patterns. The periodic pattern allows for homogeneous physical properties throughout the cancellous bone portion.

According to another embodiment of the invention, the at least one compound is a sterile or a sterilized compound such that the synthetic bone can be taken into the operating room This allows for the surgeon to double-check on the synthetic bone during a real surgery.

According to another embodiment of the invention, the synthetic bone is sterile.

According to another embodiment of the invention, the at least one compound consists of or comprises at least one polymeric, a biological and/or a bio-degradable compound.

A polymeric compound comprises or consists of a polymer.

For example, the at least one polymeric compound suitable for rapid prototyping can comprise or can consist of an organic and/or inorganic polymer, a polyurethane, an urethane, an elastomer, a paste, a plaster, a polymeric cement, and/or a plastic.

The polymeric compound can also be biodegradable.

A bio-degradable compound can be degraded by microorganisms such as fungi, bacteria, or other microorganisms.

According to another embodiment of the invention, the synthetic bone comprises or consists of a first polymeric compound composition and a second polymeric compound composition. A polymeric compound composition comprises at least one polymeric compound.

According to another embodiment of the invention, the synthetic bone comprises a plurality of polymeric compounds, wherein particularly a first polymeric compound composition comprises a first kind of polymeric compounds and the second polymeric compound composition comprises a second kind of polymeric compounds.

According to another embodiment of the invention, the first and/or second polymeric compound composition comprises a polymer or consists of a polymeric mixture.

According to another embodiment of the invention, the at least one compound or the first compound composition can be a UV-curable elastomer, particularly a rigid (after curing) acrylonitrile butadiene styrene-like compound suitable for rapid prototyping, particularly 3D-printing.

According to another embodiment of the invention, the at least one compound or the second compound composition can be a UV-curable polymer, particularly a rigid (after curing) polycarbonate-like compound, suitable for rapid prototyping, particularly 3D-printing.

According to another embodiment of the invention, the first polymeric compound composition is more elastic than the second polymeric compound composition after curing.

The first polymeric compound composition can for example be VisiJet CR-BK, and/or the second polymeric compound composition can be VisiJet CR-CL 200, both compound compositions can be obtained by 3D Systems Inc., 333 Three D Systems Circle Rock Hill, South Carolina, U.S.A.

According to another embodiment of the invention, the cortical bone portion consists of the first polymeric compound composition and the cancellous bone portion consists of the second polymeric compound composition.

The cortical bone portion of the synthetic bone can for example consist of a mixture 25%:75% of the first and second polymeric compound composition, particularly VisiJet CR-BK : VisiJet CR-CL 200.

Cortical bone portions with higher proportions of the second polymeric compound, particularly of VisiJet CR-CL 200 only, can exhibit a too rigid behavior of the cortex and lead to unrealistic insertional site preparation and fracturing of the pedicle during pedicle screw insertion. Cortical bone portions with a higher proportion of the first polymeric compound composition, particularly VisiJet CR-BK, can exhibit a too elastic behavior during insertional site preparation and laminotomy.

The cancellous bone portion can consist of the second polymeric compound composition only, particularly of VisiJet CR-CL 200 only. The cancellous bone portion comprising only the second polymeric compound composition exhibits a realistic haptic and acoustic feedback during pilot hole preparation and pedicle screw insertion. A lower proportion of VisiJet CR-CL 200 might lead to a rubber-like haptic feedback and absent acoustic feedback.

According to another embodiment of the invention, the periodic pattern is a triply symmetric, particularly minimal surface pattern, wherein the triply symmetric surface is particularly free from self-intersections.

Such intersection-free surfaces are also referred to as embedded surfaces.

A minimal surface is a surface that locally minimizes its area, particularly under predefined boundary conditions.

A triply periodic surface is a surface that repeats itself along all three dimensions.

A triply periodic minimal surface is a minimal surface that repeats itself along all three dimensions.

A synthetic bone with a periodic pattern being a non-intersecting (free of self-intersections) surface particularly divides the volume of the cancellous bone portion in two sub-volumes.

When manufacturing the synthetic bone with a rapid prototyping process, such as 3D-printing, a support material such as wax or a wax-like compound is frequently used to fill the hollow spaces in the synthetic bone, particularly the hollow spaces in the periodic pattern.

After the synthetic bone is printed and cured, the synthetic bone is heated to a temperature at which the support material melts and drains from the synthetic bone.

A periodic pattern that is not self-intersecting grants that only non-closed compartments are present in the periodic pattern, which allows the support material to drain from the periodic pattern completely.

According to another embodiment of the invention, the cortical bone portion has at least one opening, particularly two or three openings, wherein the at least one opening is particularly formed such that the support material can be drained from the synthetic bone, wherein the synthetic bone particularly comprises a cortical bone fraction that is formed as a lid or a closure for the at least one opening in the cortical bone portion, such that particularly after draining the support material from the synthetic bone, the at least one opening can be closed with the cortical bone fraction.

The cortical bone fraction can for example be glued to or into the opening such that it completely closes or particularly seals the opening.

According to another embodiment of the invention, the periodical pattern is a gyroid.

A gyroid is a specific triply symmetric minimal surface.

A gyroid can be expressed as

$$\sin(a * x) * \cos(b * y) + \sin(b * y) * \cos(c * z) + \sin(c * z) * \cos(a * x) = 0 \qquad \text{Eq. 1}$$

wherein x, y, and z are the spatial coordinates of an appropriate Cartesian coordinate system, and a, b, and c are pre-factors determining the periodicity of the gyroid along the respective direction.

The coordinates x, y, and z are particularly given in units of millimetres and the pre-factors a, b and c are particularly given in reciprocal length such as 1/mm or 1/ cm.

Equation Eq. 1 defines the overall appearance of the gyroid. Additional parameters that can be provided or adjusted are the thickness of the pattern, and its periodicity. These additional parameters cna be for example provided for or during the manufacturing process.

According to another embodiment of the invention, the periodic pattern is a gyroid with an altered periodicity, wherein the pre-factors are between

$$2\ mm^{-1} < a, b, c < 3\ mm^{-1} \text{ wherein particularly } a, b, c = 2.5\ mm^{-1}.$$

The synthetic bone with a gyroid with the abovementioned pre-factors mimics the mechanical, physical and acoustic properties of the cancellous bone portion particularly well.

However, other pre-factors might be suitable in order to mimic the cancellous bone structure of a child's bone, such as a child's vertebra or a cervical vertebra.

The periodic pattern is particularly not a molecular periodic pattern or a periodic pattern with a periodicity smaller than 100 $\mu$m. A foam is not considered to exhibit a periodic pattern.

According to another embodiment of the invention, the periodical pattern of the cancellous bone portion of the synthetic bone comprises wall portions consisting of the at least one polymeric compound or the second polymeric compound composition and void portions being filled with air, gas, a powder-like material, or a support material such as wax or a wax-like compound.

A cancellous (real) bone comprises structures that are referred to as trabecular structures. The trabecular structures are replaced by the periodic pattern in the synthetic bone, wherein the wall portions serve the same objective as the trabecular structure, while the void portions serve as the trabecular spacing between the sheet- or rod-like trabecular structures.

By increasing the thickness of the wall portions a denser cancellous bone portion can be mimicked.

By adjusting the ratio between the volume of the wall portions and the volume of the void portions the cancellous bone portion can be adjusted such that the physical, mechanical and acoustic properties of the cancellous bone portion, i.e. the periodic pattern resemble that of a real bone.

This can also be achieved by adjusting the frequency of the periodic pattern.

According to another embodiment of the invention, the wall portions of the periodic pattern make up a volume percentage in the range of 20% to 60%, particularly in the range of 25% to 45%, more particularly in the range between 30% and 40%, particularly 35% (which particularly yields an average bone as defined above) and particularly wherein the void portions comprise the corresponding other volume percentage.

A synthetic bone with a cancellous bone portion lying in the range specified in this embodiment shows good agreement to the physical, mechanical and acoustic properties of a real bone.

Furthermore, a synthetic bone having a cancellous bone portion within the range of this embodiment can mimic an osteoporotic bone, an average bone with respect to its physical properties, and a bone exhibiting an above-average hardness.

The synthetic bone according the invention can mimic real bones by their physical, mechanical and acoustic properties.

According to another embodiment of the invention, a thickness of the wall portions of the cancellous bone portion is between 0.1 mm and 0.55 mm, particularly between 0.2 mm and 0.4 mm, particularly 0.3 mm and particularly wherein the void portions fill up the corresponding rest of the periodic cancellous portion, wherein the distance between the wall portions is between 0.5 mm and 1.5 mm, particularly 1 mm.

A distance of 1 mm between the wall portions with pre-factors a = b = c = 2.5 $cm^{-1}$ and a thickness of the wall portions of 2 mm is one well-suited set of parameters for a synthetic bone according to the invention.

The periodic pattern of the cancellous bone portion with a thickness as specified in this embodiment allows for a realistic mimicking of a real bone, as particularly the physical, mechanical and acoustic properties are similar or identical to those of a real bone.

A wall thickness of the wall portion of 0.3 mm of the cancellous bone portion results in an average bone with average physical properties as defined above. The term average refers to the average or normal physical property particularly with regard to acoustic (sound), breakage, and/or insertional torque properties of a healthy real bone.

A wall thickness below 0.3 mm of the cancellous bone portion corresponds to an osteoporotic synthetic bone behavior, while a thicker wall portion yields a particularly hard synthetic bone.

According to another embodiment of the invention, the cortical bone portion has a thickness in the range of 1 mm and 3.5 mm, more particularly between 1.3 mm and 2.6 mm, particularly 1.7 mm.

In order to mimic a real bone as closely as possible, a cortical portion with a thickness in the range specified above is particularly suitable.

The thickness of the cortical portion and particularly the pedicle size i.e. the diameter of the cancellous bone inside the smallest part of the pedicle, the so-called bottleneck, particularly determines the necessary insertional torque that is needed in order to drill a surgical screw into the synthetic bone.

Moreover, the specific structure, e.g. the periodicity and/or the wall thickness of the periodic pattern of the cancellous bone portion influences the insertional torque after penetrating the cortical bone portion.

One advantage of the periodic pattern is that it provides enough void spaces such that the synthetic bone is not breaking apart when a surgical screw is inserted, while sustaining a realistic touch and feel and particularly a realistic insertional torque for surgical screws.

For example, the higher the periodicity of the periodic pattern the thicker the cortical bone portion should be in order to avoid a fracture of the synthetic bone when for example inserting a surgical screw into the pedicle portion.

Only a synthetic bone comprising the cortical bone portion in combination with the periodically structured cancellous bone portion provides the realistic and natural touch and feel properties, i.e. the correct physical properties for the synthetic bone according to the invention, while allowing the design of various different kinds of bones, such as an average bone with an average hardness or a particularly hard bone, particularly by adjusting the parameters of the periodic pattern and the cortical wall thickness.

When simulating or training a surgery, the insertional torque is an important parameter of the synthetic bone.

A synthetic bone having a cortical portion with a thickness in the range around 1.7 mm or exactly 1.7 mm yields a synthetic bone that has similar insertional torque properties to a real bone.

A thinner cortical bone portion leads to a synthetic bone that requires a lesser insertional torque and thus resembles an osteoporotic bone, while a cortical bone portion with a greater thickness than 1.7 mm yields a synthetic bone that resembles a bone being harder than the average bone.

On the other hand, the cancellous bone portion influences the insertional torque once the cortical bone portion is penetrated as laid out above such that the insertional torque can also be tuned by the structure of the periodic pattern of the cancellous bone portion.

According to another embodiment of the invention, the synthetic bone mimics a vertebra, particularly a human vertebra.

The terms "mimicking" or "resembling" particularly refer to at least one property, particularly a plurality of properties of the synthetic bone that corresponds or is very similar to its counterpart real bone. It is desirable that except the composition of the synthetic bone and the structure of the cancellous bone portion, other properties such as physical, mechanical and acoustic properties are as close as possible to the real bone properties.

Other properties such as insertional torque, elasticity, hardness, stiffness, haptic properties, weight and outer geometry of the synthetic bone should also be as close as possible to the properties of its real counterpart.

The terms "mimicking" and "resembling" refer to that objective.

Therefore, the synthetic bone resembling a vertebra refers particularly to a synthetic bone, whose appearance, i.e. its shape, but also the other abovementioned properties, are similar or identical to a vertebra.

A vertebra is particularly difficult to mimic, as it exhibits various structural components such as the vertebral foramen, the spinous process, the transverse process and the superior articular process.

All these structural components have varying physical, especially mechanical properties that have to be modeled accordingly. The synthetic bone according to the invention is particularly well suited to model the vertebra, as it is manufactureable by a rapid prototyping process, particularly 3D-printing, which is suitable to manufacture complex structures of various shape and size at comparable high resolution.

According to another embodiment of the invention, the cortical bone portion comprises a pedicle wall portion located at the corresponding location of the pedicle of the vertebra, wherein the pedicle wall portion has a thickness between 0.3 mm and 3.2 mm, particularly between 0.6 mm and 2.4 mm.

The pedicle wall portions particularly comprise two fractions, namely the medial pedicle wall portion and the lateral pedicle wall portion.

The medial pedicle wall portion can have a thickness in the range of 0.4 mm to 3.2 mm, particularly in the range of 0.8 mm to 2.4 mm.

The lateral pedicle wall portion can have a thickness in the range of 0.3 mm to 2.4 mm, particularly in the range of 0.6 mm to 1.8 mm.

The thickness of the pedicle wall portion refers particularly to the thickness of the cortical portion at the pedicle location enclosing the periodic pattern.

The pedicle portion is of particular interest in a synthetic bone or a synthetic vertebra, as in many surgical procedures on the spine, the pedicle portions are the loci where pilot holes are drilled, or surgical screws are inserted into the vertebra.

For surgical simulations of pedicle screw insertions, the pedicle wall thickness should be precisely defined and should lie in the ranges disclosed in this embodiment.

According to another aspect of the invention, a system comprises the following components:

- A plurality of synthetic bones in the form of vertebrae according to the invention,
- An elongated elastic cushion, wherein the cushion is particularly a gas-filled balloon, wherein the elastic cushion extends through the plurality of vertebrae, particularly through a plurality of vertebral foramens of the synthetic vertebrae, wherein the vertebrae are arranged such that they form at least a portion of a spine and the elastic cushion forms the spinal cord of said portion.

The synthetic vertebrae can be acquired and manufactured according to patient-specific data, e.g. from imaging methods such as computer tomography or MRI.

Each vertebra can be modeled with specific physical and mechanical properties such as to mimic the real vertebrae of a patient.

The synthetic vertebrae can be arranged on a frame that is configured to provide a fixture that allows the vertebrae to move as in a real spine.

The advantage of such a synthetic spinal column is that in a training surgery, i.e. simulated surgery on the synthetic spinal column formed by the plurality of synthetic vertebrae, the cushion can be damaged, wherein particularly if the cushion is a gas-filled balloon, the balloon pops, such that any damage to the spinal cord be assessed in training.

The problem according to the invention is also solved by a method for manufacturing a synthetic bone, particularly for manufacturing the synthetic bone according to the invention.

The method according to the invention comprises at least the following steps:

- acquiring or providing a particularly three-dimensional model of a bone, for example by a computer tomography method, a magnetic resonance imaging method or from a database;
- particularly adjusting parameters of a synthetic bone that is to be manufactured, particularly wherein said parameters comprise a thickness of a cortical portion of the synthetic bone, a density, a periodicity, or a thickness of a wall portion of a cancellous bone portion of the synthetic bone, particularly wherein the parameters are adjusted according to patient data and/or the acquired bone model;

- selecting a cortical bone portion along an outer surface or an outline of the bone model, wherein the cortical bone portion has a predefined thickness profile, such that the cortical bone portion forms an outer layer of the synthetic bone;
- selecting an enclosed volume of the cortical bone portion and arranging a periodic pattern in place of the cancellous bone portion inside this volume, wherein particularly the whole volume is filled with the periodic pattern;
- particularly generating a rapid prototyping or a 3D-printable file from the bone model with the periodic cancellous portion;
- manufacturing the synthetic bone with the cortical bone portion and the periodic cancellous portion using a rapid prototyping process, particularly a 3D-printing method.

It is noted that terms and definitions used in the embodiments of the method according to the invention are identical to the terms and definitions given for the synthetic bone and its embodiments according to the invention. For this reason, redundant definitions are omitted.

The acquisition of a particularly three-dimensional model of a bone particularly includes the receiving of data of such a model. The model can be a virtual model, i.e. saved on a computer-readable medium in a corresponding file format.

The bone model can be stored on a non-transitory or a transitory computer-readable medium and can be retrieved from such a medium.

The bone model does not need to be a copy of a real bone, but it can also be a model of a generic bone.

It is also possible to acquire the bone model from a real bone for example by means of computer tomography or a magnetic resonance imaging method. Also other imaging methods can be used such as for example positron emission tomography.

The acquisition of the bone model can comprise the execution of computer programs and software that are configured to filter or select specific parts of the bone model, and/or that are configured to extract or segment specific parts of the bone model, particularly from a dataset comprising the bone model.

In order to manufacture a synthetic bone, the outline of the bone model is estimated and a cortical bone portion is assigned to the outline. The cortical bone portion is particularly formed as a layer with a predefined thickness profile.

The thickness profile can be adjusted in thickness, in order to adjust the physical and mechanical properties of the synthetic bone.

The estimation of the outline of the bone model can comprise actions such as segmenting, extracting and/or selecting regions of the bone model.

The synthetic bone and particularly the thickness of the cortical portion can be adapted to a patient's anatomy, such that the physical and mechanical bone properties of the synthetic bone match the physical and mechanical properties of the real bone of the patient.

The cortical bone portion encloses a volume that is filled with a cancellous bone portion that is a periodic pattern.

The cancellous bone portion can be adapted to a patient's anatomy, such that the physical and mechanical bone properties of the synthetic bone match the patient's bone properties.

The cancellous bone portion is particularly adapted by adjusting the periodicity of the periodic pattern, the wall thickness of the periodic pattern and/or the void-to-mass ratio of the periodic pattern.

In case the bone model is a virtual model, the insertion of the periodic cancellous bone is a virtual replacement, too.

In certain embodiments it might be advantageous to generate a specific file format, for example a stereolithography file format (STL) or a 3D manufacturing format (3MF) used for 3D-printing from the bone model with the periodic pattern.

Subsequently, the synthetic bone is manufactured for example by a 3D-printer or another rapid prototyping device, wherein the synthetic bone comprises the periodic cancellous bone portion and the cortical bone portion with the predefined thickness profile.

The method according to the invention allows for flexible manufacturing of a synthetic bone with realistic bone properties, while the processing steps that are required to arrive at the synthetic bone are comparably simple, and can be implemented in an automated fashion, by adjusting a selected set of parameters.

This way, any patient-specific bone property can be modeled in the synthetic bone. Such a patient-specific synthetic bone allows for surgical training for particularly patient-specific surgery.

Using a rapid prototyping method, particularly a 3D-printing method, allows for manufacturing the synthetic bone with a sufficiently high spatial resolution, which other manufacturing methods do not provide.

Particularly, as the periodic pattern can have a periodicity that is below one millimeter, it is advantageous to use a rapid prototyping method such as 3D-printing.

It is noted that the cancellous bone portion cannot be structurally resolved by conventional imaging methods. It is thus advantageous to replace the (unresolved) cancellous bone portion with the periodic pattern with a specified periodicity and wall thickness.

In turn, solely providing the acquired bone model to a rapid prototyping process would not yield a satisfactory result in terms of property matching to the real bone, as the spatial resolution of the acquisition method might not provide sufficient structural details for the cancellous bone portion.

The method according to the invention furthermore allows a cost-efficient prototyping of various bones, exhibiting various but still realistic bone properties, such as hardness, weight, insertional torque, elasticity, acoustic properties, and/or haptic properties such as touch and feel.

According to another embodiment of the invention, the synthetic bone is manufactured using at least one polymeric compound, wherein particularly the completed synthetic bone comprises only the at least one polymeric compound or one or more polymeric compositions.

According to another embodiment of the invention, the synthetic bone comprises a cortical bone portion forming the cortex of the synthetic bone and a cancellous portion, wherein the cancellous bone portion consists of a periodic pattern.

The cortical bone portion particularly completely encloses the cancellous portion of the synthetic bone, particularly once the manufacturing of the synthetic bone is completed.

According to another embodiment of the invention, the periodic pattern is manufactured as a triply symmetric particularly minimal surface pattern or a gyroid.

The specific forms and properties of the triply symmetric surface patterns and suitable gyroids are disclosed in the embodiments related to the synthetic bone and are applicable to the method as well.

According to another embodiment of the invention, the bone model is acquired from a vertebra, particularly a human vertebra.

Therefore, the resulting manufactured synthetic bone resembles or mimics a vertebra, particularly a human vertebra.

According to another embodiment of the invention, the rapid prototyping process is one of:

- a 3D-printing method, particularly using at least one polymeric compound as a printing compound,
- a fused deposition modeling method,
- a multi-jet modeling method, and/or
- a selective laser sintering method.

All these rapid prototyping processes can be used with polymers or polymeric compounds. Specific and suitable polymeric compounds and polymeric compound compositions are given in the embodiments concerning the synthetic bone and are applicable to the manufacturing as well.

According to another embodiment of the invention, the synthetic bone is manufactured from at least one polymeric compound.

This allows the use of comparably cost-effective printing techniques, such as 3D-printing.

According to another embodiment of the invention, the void portions are filled with a support material such as wax or a wax-like compound during manufacturing.

According to another embodiment of the invention, the support material is drained from the periodic pattern, particularly by heating the synthetic bone, particularly after the cancellous bone portion is completed.

The cortical bone portion can have an opening through which the support material can be drained.

The opening can be closed in a subsequent processing step by the cortical bone fraction that complements said opening.

According to another embodiment of the invention, a cortical bone portion of the synthetic bone is manufactured as an outer and denser layer of the synthetic bone, wherein the outer layer has a predefined but adaptable thickness profile.

The thickness profile can be adjusted according to patient data or a desired resulting bone property.

The method according to the invention can comprise the following steps:

- acquiring an image stack by means of an imaging technique, comprising the bone from which a synthetic bone should be manufactured;
- segmenting the image stack and extract a three-dimensional bone model of the bone from which the synthetic bone is to be manufactured;
- identifying the outline of the bone model or the thickness of a cortical bone portion in the bone model;
- assign a cortical bone portion to the outline of the bone model, the cortical bone portion having a predefined thickness profile;
- in the bone model, selecting at least one opening in the cortical bone portion and a corresponding closure for the at least one opening;
- filling a volume enclosed by the cortical bone portion with a periodic pattern;
- manufacturing the synthetic bone and the closure, e.g. by a 3D-printing method;
- dewaxing the manufactured synthetic bone and the closure;
- closing the opening of the cortical bone portion with the corresponding closure.

In the following, examples and embodiments of the invention are described by means of a figure description. It is shown in

Fig. 1     two three-dimensional views of a synthetic bone;
Fig. 2     a cross-sectional view of a bone model;
Fig. 3     a three-dimensional view of the periodic cancellous bone portion;
Fig. 4     a gyroid pattern;
Fig. 5     a diagram illustrating the method according to the invention; and
Fig. 6     a synthetic spine formed by a plurality of synthetic vertebra.

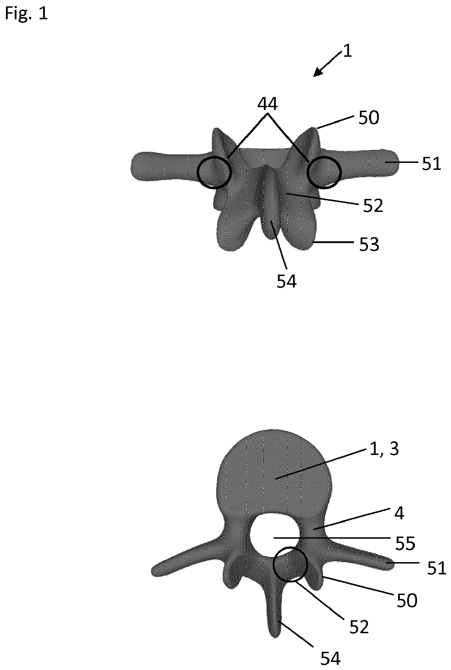

In Fig. 1 two three-dimensional views of a synthetic bone 1 according to the invention are shown. The top panel shows a side view of the synthetic bone 1, wherein the bottom panel shows a top view onto the synthetic bone 1.

The synthetic bone 1 is a synthetic vertebra. The vertebra has various processes, such as the superior articular process 50, the transverse process 51, the inferior articular process 53 and the spinous process 54. Furthermore, the lamina 52 of the vertebra can be seen.

Of particular interest of the synthetic bone 1 is a cortical bone portion 3 that comprises the pedicle wall portions 4. Surgical screws during spinal surgery are oftentimes inserted along the pedicle. Therefore, the precise modeling of the pedicle wall portion 4 is important. In the upper panel of Fig. 1, the pedicle screw insertion sides 44, where surgical screws are inserted into the vertebra, are marked with circles. The synthetic bone 1 comprises an outer layer which represents the cortical bone portion 3.

The cortical bone portion 3 of the synthetic bone 1 is made from a first polymeric compound composition with hardness, elasticity and haptic properties identical to the hardness and the synthetic properties of a real vertebra. For this reason the cortical bone portion 3, particularly the pedicle wall portions 4 might exhibit different thicknesses along the synthetic bone 1.

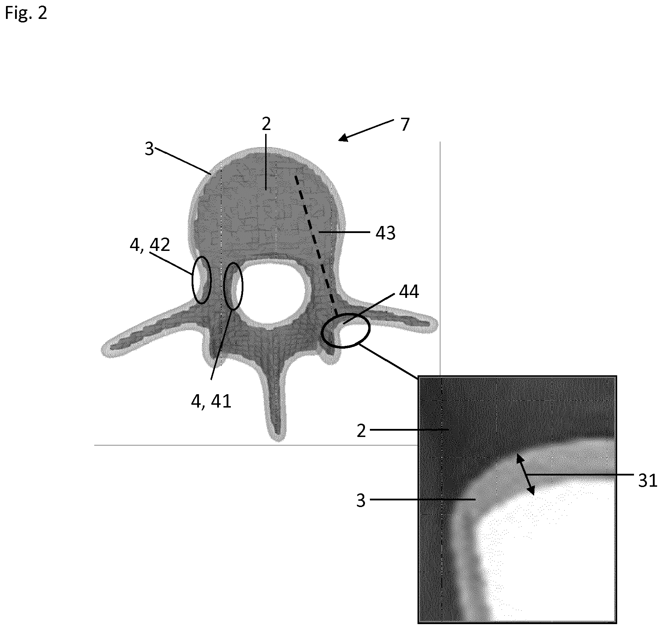

The cortical bone portion 3 of the synthetic bone 1 encloses the cancellous bone portion 2. The cancellous bone portion 2 can be seen in the cross-sectional view the vertebra in Fig. 2.

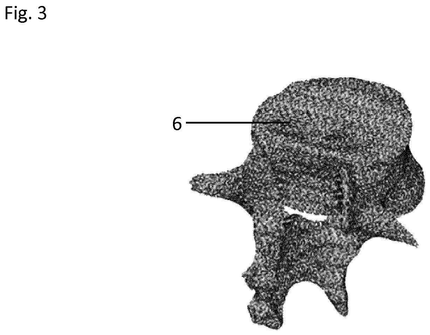

In Fig. 2 a bone model 7 is shown. To arrive at the synthetic bone 1 from the bone model 7, the cancellous bone portion 2 has to be replaced by a periodic pattern 6 (cf. e.g. Fig. 3). In Fig. 2 the pedicle screw insertion site 44 is marked by a circle along with the pedicle screw trajectory 43 (broken line). It is differentiated between the lateral pedicle wall portion 42 and the medial pedicle wall portion 41. These two pedicle wall portions 4 can exhibit different thicknesses in the synthetic bone 1, as well as in the real bone (cf. e.g. Table 1).

The synthetic bone 1 consists of the cortical bone portion 3 which is made from the first polymeric compound composition and the periodic pattern 6 (not shown in Fig. 2) that replaces the cancellous bone portion 2.

Fig. 2 comprises a zoomed-in box, highlighting the cortical bone portion 3 layer and its thickness 31.

Figure 3 shows the periodic pattern 6 that is used to replace the cancellous bone portion 2 of the acquired bone model 7 (see Fig. 2). The periodic pattern 6 consists of a second polymeric compound composition that is more rigid than the first polymeric compound composition.

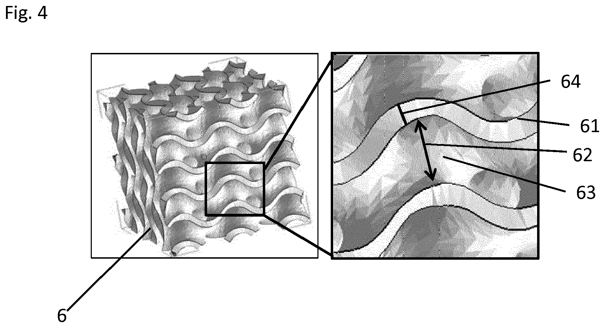

In the present example, the periodic pattern 6 is a gyroid. A detailed view of a gyroid is shown in Fig. 4.

The gyroid comprises wall portions 61 and void portions 63 that form the periodic pattern 6. The wall portions 61 have a predefined thickness 64 and distance 62 from each other in order to provide the cancellous bone portion 2 of the synthetic bone 1 with the appropriate physical properties. The wall portions 61 do not self-intersect.

In this example the gyroid is represented by

$$\sin(2.5 * x) * \cos(2.5 * y) + \sin(2.5 * y) * \cos(2,5 * z) + \sin(2.5 * z) * \cos(2.5 * x) = 0$$

In many imaging techniques the structure of the cancellous bone portion 2 cannot be structurally resolved. The synthetic bone 1 with the periodic pattern 6 that replaces the unresolved cancellous bone portion 2 of the bone model 7 solves this problem and allows the manufacturing of a realistic synthetic bone 1 with rapid prototyping techniques.

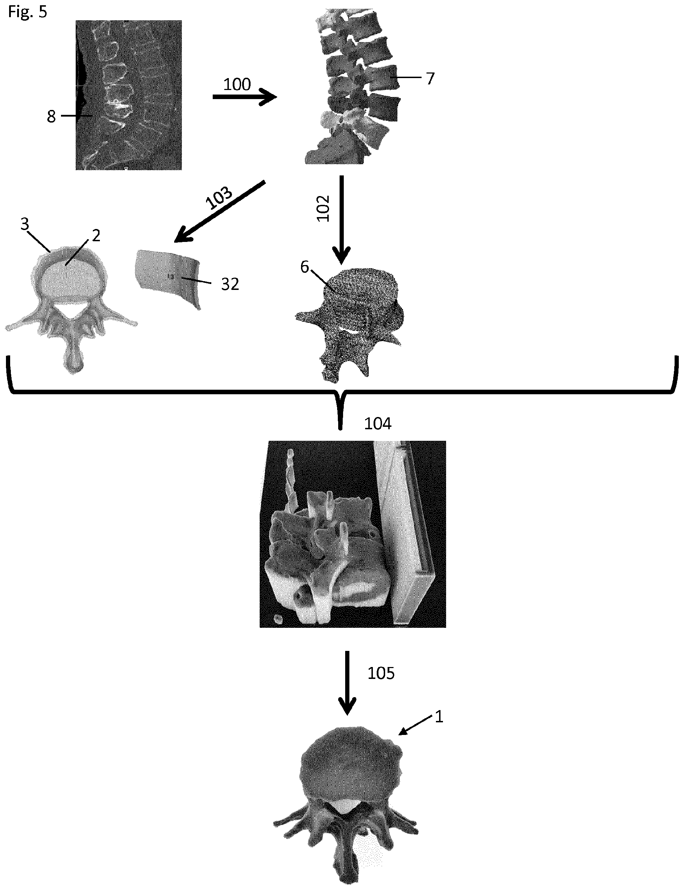

In Fig. 5 the method according to the invention is depicted schematically. First, the bone model 7 is acquired 100 by means of computer tomography. The bone model 7 is acquired by recording a stack of images 8 that are subsequently processed such that a segmentation of the bone model 7 is achieved. From the segmented images a three-dimensional bone model 7 from e.g. at least one vertebra is acquired.

In a next step, the cortical bone portion 3 is identified 103 and selected for example by identifying the outer contour of the acquired bone model 7 and providing a layer of a thickness profile for the cortical bone portion 3. Furthermore, a closure for an opening 32 in the cortical bone portion 3 is selected.

The cancellous bone portion 2 enclosed by the cortical bone portion 3 is replaced 102 in the bone model 7 by the periodic pattern 6, here a gyroid. The bone model 7 with the periodic pattern 6 is submitted 104 to a 3D-printing method that uses polymeric compounds in order to print the synthetic bone 1.

In order to stabilize the synthetic bone 1 during printing, a support material such as a wax or a wax-like compound is used and incorporated into the synthetic bone 1.

Once the synthetic bone 1 is printed, the synthetic bone is heated 105 such that the wax or the wax-like compound drains through the opening in the cortical bone portion 3.

Once the wax has been drained, the closure 32 is glued to the opening, such that a complete synthetic bone 1 is achieved.

The closure 32 and the opening are arranged preferentially at locations on the synthetic bone 1 where no (simulated) surgery is planned.

With the method according to the invention L3-vertebra models can be constructed for surgical assessment. Five different 3D-printed vertebra models with different proportions in cortical bone portion and wall portion thickness of the periodic pattern have been assessed. Three of these five models are considered to resemble a real bone in regard to biomechanical behaviour during performance of surgical procedures (e.g. pedicle screw insertion).

One model was rated as a representative model for osteoporotic to normal bone, one for normal to hard bone and one for a hard bone.

The detailed parameters for the five models are given in Table 1:

Table 1:

| | Thickness of wall portion of the periodic pattern [mm] | Cortical bone portion thickness [mm] | Medial pedicle wall portion thickness [mm] | Lateral pedicle wall portion thickness [mm] | Density of periodic pattern / cancellous bone portion [%] | Result |
|---|---|---|---|---|---|---|
| **3D-Model 1** | 0.15 | 1.1 | 0.4 | 0.3 | 17.8 | too soft |
| **3D-Model 2** | 0.2 | 1.3 | 0.8 | 0.6 | 24.7 | osteoporotic to normal |
| **3D-Model 3** | 0.3 | 1.7 | 1.6 | 1.2 | 34.6 | normal to hard |
| **3D-Model 4** | 0.4 | 2.6 | 2.4 | 1.8 | 45.5 | hard |

(continued)

|  | Thickness of wall portion of the periodic pattern [mm] | Cortical bone portion thickness [mm] | Medial pedicle wall portion thickness [mm] | Lateral pedicle wall portion thickness [mm] | Density of periodic pattern / cancellous bone portion [%] | Result |
|---|---|---|---|---|---|---|
| 3D-Model 5 | 0.5 | 3.4 | 3.2 | 2.4 | 55.5 | too hard |

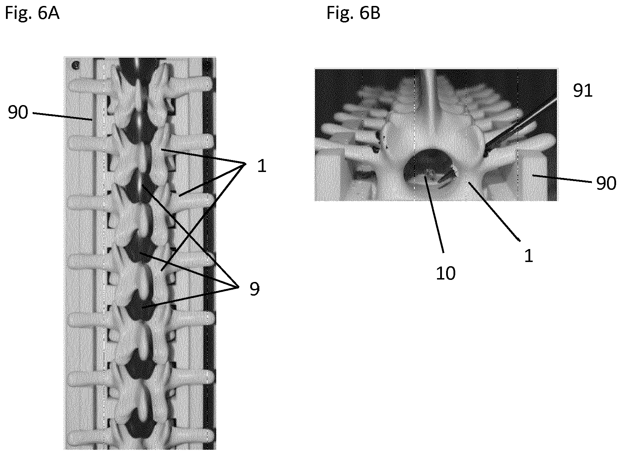

In Fig. 6A a synthetic spinal column comprising a plurality of synthetic vertebrae 1 according to the invention and a gas-filled balloon 9 that extends through all vertebrae 1 are depicted and form the spinal cord.

The vertebrae 1 are arranged on a frame 90 that allows the vertebrae 1 to perform movements according to a real spine.

In Fig. 6B a training of a spinal surgery on a synthetic spine is shown, wherein the perforation of a vertebra 1 is shown that made the balloon 9 burst. The trajectory of the surgical tool 91 was inappropriate and would have led to a spinal cord injury of the patient during surgery.

A spine or a spine portion comprising a plurality of synthetic vertebrae 1 according to the invention and the gas-filled balloon 9 provides a realistic patient-specific model for surgical training.


**Claims**

1.    Synthetic mammalian bone (1), wherein the synthetic bone (1) comprises at least one compound that is suitable for rapid prototyping, wherein the synthetic bone (1) comprises a cortical bone portion (3) and a cancellous bone portion (2), wherein the cortical bone portion (3) forms a layer enclosing the cancellous bone portion (2), wherein the cortical bone portion (3) exhibits a higher density than the cancellous bone portion (2), and wherein the cancellous bone portion (2) is formed as a periodic pattern (6).

2.    Synthetic bone according to claim 1, wherein the synthetic bone (1) comprises or consists of the at least one compound, wherein the at least one compound is a polymeric, a biological or a biodegradable compound.

3.    Synthetic bone according to claim 1 or 2, wherein the cortical bone portion (3) consists of a first polymeric compound composition and the periodic pattern (6) consists of a second polymeric compound composition.

4.    Synthetic bone, according to one of the preceding claims, wherein the periodic pattern (6) is a triply symmetric surface pattern or a gyroid.

5.    Synthetic bone according to one of the preceding claims, wherein the periodic pattern (6) of the cancellous bone portion (2) comprises gas-filled void portions (63) and wall portions (61) consisting of the at least one compound and, particularly wherein the wall portions (61) are free of self-intersections.

6.    Synthetic bone according to claim 5, wherein the wall portions (61) of the cancellous bone portion (2) make up a volume percentage in the range of 20% to 60%.

7.    Synthetic bone according to claim 5 or 6, wherein the thickness (64) of the wall portions (61) of the cancellous bone portion (2) is between 0.1 mm and 0.55 mm.

8.    Synthetic bone according to one of the preceding claims, wherein the synthetic bone (1) is a synthetic vertebra, particularly a synthetic human vertebra.

9.    A synthetic spinal column portion comprising the components:

- a plurality of synthetic bones (1) in form of synthetic vertebrae according to one of the preceding claims,
- an elongated elastic cushion (9), wherein the cushion (9), wherein the elastic cushion (9) extends through the plurality of synthetic vertebrae (1), wherein the vertebrae (1) are arranged such that they form at least a portion of a spine and the elastic cushion (9) forms the spinal cord of said portion.

10. Method for manufacturing a synthetic bone (1), particularly a synthetic bone (1) according to one of the claims 1 to 8, comprising the steps of:

- acquiring (100) a bone model (7);
- selecting (103) an outline of the bone model (7);
- selecting a cortical bone portion with a predefined thickness profile along the outline of the bone model;
- filling the volume enclosed by the cortical bone portion (102) with a periodic pattern (6), yielding a periodic cancellous bone portion (2);
- manufacturing (104, 105) the synthetic bone (1) with the cortical portion and with the periodic cancellous bone portion (6) using a rapid prototyping process.

11. Method according to claim 10, wherein the periodic pattern (6) is a triply symmetric, particularly minimal surface pattern or a gyroid.

12. Method according to one of the claims 10 to 11, wherein the bone model (7) is acquired from a vertebra, particularly from a human vertebra.

13. Method according to one of the claims 10 to 12, wherein the rapid prototyping process (104, 105) is a 3D-printing method.

14. Method according to one of the claims 10 to 13, wherein the synthetic bone (1) is manufactured from at least one polymeric or at least one biological compound.

15. Method according to one of the claims 10 to 14, wherein a support material is drained (105) from the periodic pattern (6) through an opening in the cortical bone portion (3), particularly wherein the opening is closed with a corresponding cortical bone fraction (32).

Fig. 1

Fig. 2

Fig. 3

Fig. 4

6

64
61
62
63

Fig. 5

Fig. 6A

Fig. 6B

Europäisches
Patentamt

European
Patent Office

Office européen
des brevets

# EUROPEAN SEARCH REPORT

Application Number

EP 18 15 9979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHENGFA WANG: "Lightweight of Artificial Bone Models Utilizing Porous Structures and 3D Printing", INTERNATIONAL JOURNAL OF PERFORMABILITY ENGINEERING, 1 January 2017 (2017-01-01), XP55503478, DOI: 10.23940/ijpe.17.05.p8.633642 * page 633 - page 634; figure 7 * | 1-15 | INV. A61L27/50 A61L27/56 G09B23/28 G09B23/30 G09B23/34 |
| X | DONG J YOO: "Porous scaffold design using the distance field and triply periodic minimal surfacemodels", BIOMATERIALS, ELSEVIER SCIENCE PUBLISHERS BV., BARKING, GB, vol. 32, no. 31, 7 July 2011 (2011-07-07), pages 7741-7754, XP028268192, ISSN: 0142-9612, DOI: 10.1016/J.BIOMATERIALS.2011.07.019 [retrieved on 2011-07-14] * page 7742 - page 7743; figure 16 * | 1-4,8, 10-14 | |
| X | AFSHAR M ET AL: "Additive manufacturing and mechanical characterization of graded porosity scaffolds designed based on triply periodic minimal surface architectures", JOURNAL OF THE MECHANICAL BEHAVIOR OF BIOMEDICAL MATERIALS, ELSEVIER, AMSTERDAM, NL, vol. 62, 28 May 2016 (2016-05-28), pages 481-494, XP029641488, ISSN: 1751-6161, DOI: 10.1016/J.JMBBM.2016.05.027 * pages 482, 493; figures * | 1-4,10, 11,13,14 | TECHNICAL FIELDS SEARCHED (IPC) A61L G09B |
| A | US 2017/263158 A1 (EAST JOHNNY WAYNE [US] ET AL) 14 September 2017 (2017-09-14) * claims; figures * | 1-15 | |

-/--

The present search report has been drawn up for all claims

| Place of search | Date of completion of the search | Examiner |
|---|---|---|
| The Hague | 31 August 2018 | Espinosa y Carretero |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 18 15 9979

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/193776 A1 (THURNER MARC [CH] ET AL) 10 July 2014 (2014-07-10) * paragraphs [[0016]], [[0041]]; claims * | 1-15 | |
| A | US 6 993 406 B1 (CESARANO III JOSEPH [US] ET AL) 31 January 2006 (2006-01-31) * columns 1, 5, 7; claims * | 1-15 | |
| A | YÁNEZ A ET AL: "Gyroid porous titanium structures: A versatile solution to be used as scaffolds in bone defect reconstruction", MATERIALS & DESIGN, ELSEVIER, AMSTERDAM, NL, vol. 140, 23 November 2017 (2017-11-23), pages 21-29, XP085414273, ISSN: 0264-1275, DOI: 10.1016/J.MATDES.2017.11.050 * abstract * | 1-15 | |

TECHNICAL FIELDS
SEARCHED      (IPC)

The present search report has been drawn up for all claims

| Place of search | Date of completion of the search | Examiner |
|---|---|---|
| The Hague | 31 August 2018 | Espinosa y Carretero |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 15 9979

This annex lists the patent family members relating to the patent documents cited in the above-mentioned European search report.
The members are as contained in the European Patent Office EDP file on
The European Patent Office is in no way liable for these particulars which are merely given for the purpose of information.

31-08-2018

| Patent document cited in search report | | Publication date | Patent family member(s) | | Publication date |
|---|---|---|---|---|---|
| US 2017263158 | A1 | 14-09-2017 | NONE | | |
| US 2014193776 | A1 | 10-07-2014 | CH | 705356 A2 | 15-02-2013 |
| | | | CN | 103764054 A | 30-04-2014 |
| | | | EP | 2741700 A1 | 18-06-2014 |
| | | | JP | 6352183 B2 | 04-07-2018 |
| | | | JP | 2014522710 A | 08-09-2014 |
| | | | US | 2014193776 A1 | 10-07-2014 |
| | | | WO | 2013021000 A1 | 14-02-2013 |
| US 6993406 | B1 | 31-01-2006 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016115625 A1